(19) Europäisches Patentamt
European Patent Office
Office européen des brevets

(11) EP 4 296 290 A1

# (12) EUROPEAN PATENT APPLICATION
published in accordance with Art. 153(4) EPC

(43) Date of publication:
**27.12.2023 Bulletin 2023/52**

(21) Application number: **22775135.1**

(22) Date of filing: **09.03.2022**

(51) International Patent Classification (IPC):
***C08F 290/00*** (2006.01) ***B33Y 10/00*** (2015.01)
***B33Y 80/00*** (2015.01) ***B29C 64/112*** (2017.01)
***B29C 64/124*** (2017.01) ***B29C 64/314*** (2017.01)
***B29C 64/343*** (2017.01) ***B33Y 40/00*** (2020.01)
***B33Y 70/00*** (2020.01)

(52) Cooperative Patent Classification (CPC):
**B29C 64/112; B29C 64/124; B29C 64/314; B29C 64/343; B33Y 10/00; B33Y 40/00; B33Y 70/00; B33Y 80/00; C08F 290/00**

(86) International application number:
**PCT/JP2022/010410**

(87) International publication number:
**WO 2022/202343 (29.09.2022 Gazette 2022/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.03.2021 JP 2021049004**
**13.01.2022 JP 2022003891**

(71) Applicant: **MITSUI CHEMICALS, INC.**
**Tokyo 104-0028 (JP)**

(72) Inventors:
- **MURAI, Hiroki**
**Sodegaura-shi, Chiba 299-0265 (JP)**
- **SAKAMAKI, Toshikazu**
**Sodegaura-shi, Chiba 299-0265 (JP)**
- **KIMURA, Mai**
**Sodegaura-shi, Chiba 299-0265 (JP)**
- **ENDO, Suguru**
**Sodegaura-shi, Chiba 299-0265 (JP)**

(74) Representative: **Hoffmann Eitle**
**Patent- und Rechtsanwälte PartmbB**
**Arabellastraße 30**
**81925 München (DE)**

## (54) COMPOSITION, OPTICALLY MOLDED ARTICLE, AND DENTAL PRODUCT

(57) Provided is a composition which contains a urethane (meth)acrylate, and at least one selected from the group consisting of 2-hydroxyethyl acrylate and hydroxypropyl acrylate, in which at least one of the following (1) or (2) is satisfied: (1) a content of 2-hydroxyethyl acrylate is 10,000 ppm by mass or less with respect to a total mass of the composition; (2) a content of hydroxypropyl acrylate is 25,000 ppm by mass or less with respect to the total mass of the composition.

EP 4 296 290 A1

## Description

Technical Field

The present disclosure relates to a composition, a stereolithography product, and a dental product.

Background Art

In recent years, dental prostheses, instruments used in the oral cavity, and the like have become known as dental products, and various materials have been developed according to their respective applications.

In particular, from the viewpoint of the efficiency of molding these dental products, a method for manufacturing a three-dimensional object such as a dental product by stereolithography using a 3D printer is known.

For example, Patent Literature 1 describes a method for manufacturing a synthetic material part in which a three-dimensional printing technique is used, a base material is prepared, a material for at least one synthetic material part is applied in layers on the base material via a nozzle configuration, and curing is performed after application of one or more layers and before application of another layer, which is a method in which the layer is formed of a polymerizable synthetic material having a viscosity such that processing of the synthetic material is carried out in a non-polymerized state by means of a nozzle configuration, and this layer is polymerized away from the nozzle configuration, and is a method for manufacturing a dental prosthesis in which this method is used particularly for manufacturing a dental treatment material such as a denture base material, and a differently colored material or multiple materials are used in the manufacturing.

Patent Literature 1: Japanese Patent No. 4160311

SUMMARY OF INVENTION

Technical Problem

A stereolithography product (for example, a three-dimensional object) obtained by stereolithography using a photocurable composition may be used for dental applications, for example.

In a case in which a three-dimensional object obtained by stereolithography using a photocurable composition is used for dental applications, antibacterial properties and biocompatibility may be required.

An object of an embodiment of the present disclosure is to provide a composition capable of obtaining a stereolithography product having excellent antibacterial properties and biocompatibility, a stereolithography product manufactured using the composition, and a dental product.

Solution to Problem

Means for solving the above problems include the following aspects.

<1> A composition containing a urethane (meth)acrylate, and at least one selected from the group consisting of 2-hydroxyethyl acrylate and hydroxypropyl acrylate, in which at least one of the following (1) or (2) is satisfied:

(1) a content of 2-hydroxyethyl acrylate is 10,000 ppm by mass or less with respect to a total mass of the composition;
(2) a content of hydroxypropyl acrylate is 25,000 ppm by mass or less with respect to the total mass of the composition.

<2> The composition described in <1>, in which the content of 2-hydroxyethyl acrylate is 5 ppm by mass or more with respect to the total mass of the composition.
<3> The composition described in <1> or <2>, in which the content of 2-hydroxyethyl acrylate is 4,000 ppm by mass or less with respect to the total mass of the composition.
<4> The composition described in any one of <1> to <3>, in which the content of hydroxypropyl acrylate is 10 ppm by mass or more with respect to the total mass of the composition.
<5> The composition described in any one of <1> to <4>, in which the content of hydroxypropyl acrylate is 15,000 ppm by mass or less with respect to the total mass of the composition.
<6> The composition described in any one of <1> to <5>, in which the urethane (meth)acrylate contains a urethane (meth)acrylate having 2 or less functional groups.
<7> The composition described in any one of <1> to <6>, further containing a photopolymerization initiator.

<8> The composition described in <7>, further containing a (meth)acrylic monomer (E) other than the urethane (meth)acrylate, the 2-hydroxyethyl acrylate, and the hydroxypropyl acrylate.
<9> The composition described in <7> or <8>, in which IC50 of a cured product of the composition is 5% or more.
<10> The composition described in any one of <7> to <9>, in which a content of a bifunctional (meth)acrylate and a monofunctional (meth)acrylate is 80% by mass or more with respect to a total mass of (meth)acrylate in the composition.
<11> The composition described in any one of <7> to <10>, in which a content of the urethane (meth)acrylate is 25% by mass or more with respect to the total mass of the composition.
<12> The composition described in any one of <1> to <6>, in which a content of the urethane (meth)acrylate is 95% by mass or more with respect to the total mass of the composition.
<13> The composition described in any one of <7> to <11>, which is used for stereolithography.
<14> The composition described in any one of <1> to <13>, which is used in manufacturing a dental product.
<15> A stereolithography product of the composition according to any one of <7> to <11>.
<16> The stereolithography product according to <15>, in which the IC50 is 5% or more.
<17> A dental product including the stereolithography product according to <15> or <16>.

Advantageous Effects of Invention

According to an embodiment of the present disclosure, it is possible to provide a composition capable of obtaining a stereolithography product having excellent antibacterial properties and biocompatibility, a stereolithography product manufactured using the composition, and a dental product.

DESCRIPTION OF EMBODIMENTS

In the present disclosure, the numerical ranges indicated using "to" include the numerical values before and after "to" as the minimum and maximum values, respectively.
In the numerical ranges described step by step in the disclosure, the upper limit value or the lower limit value of one numerical range may be replaced with the upper limit value or the lower limit value of another numerical range described step by step. Moreover, in the numerical ranges described in the disclosure, the upper limit value or the lower limit value of the numerical ranges may be replaced with the values shown in the examples.
In the disclosure, "(meth)acryloyl" means acryloyl or methacryloyl, and "(meth)acrylate" means acrylate or methacrylate.
In the disclosure, "iso(thio)cyanate" means isocyanate or isothiocyanate.
In the present disclosure, the "urethane bond" includes, for example, a bond formed by reaction between an isocyanate group and a hydroxyl group and a bond formed by reaction between an isothiocyanate group and a hydroxyl group.
In the disclosure, in a case in which there are a plurality of types of substances corresponding to each component in the composition, the amount of each component in the composition means the total amount of the plurality of types of substances present in the composition unless otherwise specified.

<<Composition>>

A composition of the disclosure contains a urethane (meth)acrylate, and at least one selected from the group consisting of 2-hydroxyethyl acrylate and hydroxypropyl acrylate, and at least one of the following (1) or (2) is satisfied:

(1) a content of 2-hydroxyethyl acrylate is 10,000 ppm by mass or less with respect to a total mass of the composition;
(2) a content of hydroxypropyl acrylate is 25,000 ppm by mass or less with respect to the total mass of the composition.

The details of specific examples, preferable specific examples, preferable aspects and the like in the urethane (meth)acrylate, the 2-hydroxyethyl acrylate, the hydroxypropyl acrylate, the content of the 2-hydroxyethyl acrylate, and the content of the hydroxypropyl acrylate are the same as the details of specific examples, preferable specific examples, preferable aspects and the like in the urethane (meth)acrylate, the 2-hydroxyethyl acrylate, the hydroxypropyl acrylate, the content of the 2-hydroxyethyl acrylate, and the content of the hydroxypropyl acrylate described in the section of the first embodiment or the second embodiment which will be described later.
The composition of the present disclosure may contain a (meth)acrylic monomer (E) (hereinafter also referred to as a (meth)acrylic monomer (E)) other than urethane (meth)acrylate, 2-hydroxyethyl acrylate, and hydroxypropyl acrylate.
The details of specific examples, preferable specific examples, preferable aspects and the like of the (meth)acrylic monomer (E) are the same as the details of specific examples, preferable specific examples, preferable aspects and the like corresponding to (meth)acrylic monomers other than urethane (meth)acrylate, 2-hydroxyethyl acrylate, and hydroxypropyl acrylate described in the section of the (meth)acrylic monomer (C) in the first embodiment.

The composition of the present disclosure includes the first embodiment and the second embodiment. That is, the details of each configuration, the preferable aspect of each configuration, and the like described in the sections of the first embodiment and the second embodiment are also the details of each configuration, the preferable aspect of each configuration, and the like in the composition of the disclosure.

Each embodiment will be described below.

[First Embodiment]

<<Composition>>

The composition of the first embodiment contains urethane (meth)acrylate and 2-hydroxyethyl acrylate, and the content of the 2-hydroxyethyl acrylate is 10,000 ppm by mass or less with respect to the total mass of the composition.

Since the composition of the first embodiment contains a combination of urethane (meth)acrylate and 2-hydroxyethyl acrylate, and the content of 2-hydroxyethyl acrylate is within the above range, accordingly, a stereolithography product having excellent antibacterial properties and biocompatibility can be obtained.

The composition of the first embodiment may be composed of only urethane (meth)acrylate and 2-hydroxyethyl acrylate, or may further contain components other than urethane (meth)acrylate and 2-hydroxyethyl acrylate.

<Urethane (Meth)acrylate>

The composition of the first embodiment contains a urethane (meth)acrylate.

A cured product can be obtained by polymerizing and curing the urethane (meth)acrylate in the first embodiment.

In the first embodiment, a composition in which the content of the urethane (meth)acrylate is 90% by mass or more with respect to the total mass of the composition is also referred to as a "urethane (meth)acrylate composition".

The urethane (meth)acrylate preferably includes a urethane (meth)acrylate having 2 or less functional groups, and more preferably contains a urethane (meth)acrylate having 2 functional groups.

For example, the "urethane (meth)acrylate having two functional groups" means a urethane (meth)acrylate having two (meth)acryloyl groups.

In the first embodiment, the urethane (meth)acrylate having one functional group may be referred to as a monofunctional urethane (meth)acrylate, and the urethane (meth)acrylate having two functional groups may be referred to as a bifunctional urethane (meth)acrylate.

The urethane (meth)acrylate in the first embodiment can be used without particular limitation as long as the urethane (meth)acrylate is a compound containing a urethane bond and a (meth)acrylate group.

For example, the urethane (meth)acrylate in the first embodiment may be a reaction product of an iso(thio)cyanate compound and a hydroxy (meth)acrylate compound containing a hydroxy group and a (meth)acryloyloxy group.

In addition, the urethane (meth)acrylate in the first embodiment may be a reaction product of an isocyanate compound, a hydroxy (meth)acrylate compound containing a hydroxy group and a (meth)acryloyloxy group, and a compound other than these compounds (for example, a thiol compound containing a mercapto group).

<Iso(thio)cyanate Compound>

Examples of the iso(thio)cyanate compound include a monofunctional iso(thio)cyanate compound and a di- or higher functional iso(thio)cyanate compound.

The iso(thio)cyanate compounds may be used singly or in combination of two or more kinds thereof.

Examples of monofunctional iso(thio)cyanate compounds include 4-ethylphenyl iso(thio)cyanate, benzenesulfonyl iso(thio)cyanate, 2-(trifluoromethyl)phenyl iso(thio)cyanate, 2,6-dimethylphenyl iso(thio)cyanate, phenyl iso(thio)cyanate, hexyl iso(thio)cyanate, 2-methoxyphenyl iso(thio)cyanate, 3-methoxyphenyl iso(thio)cyanate, 4-methoxyphenyl iso(thio)cyanate, 2-ethoxyphenyl iso(thio)cyanate, 3-ethoxyphenyl iso(thio)cyanate, 4-ethoxyphenyl iso(thio)cyanate, 2-biphenyl iso(thio)cyanate, 1-naphthyl iso(thio)cyanate, 3-(triethoxysilyl)propyl iso(thio)cyanate, 2-iso(thio)cyanatoethyl methacrylate, 2-iso(thio)cyanatoethyl acrylate, cyclohexyl iso(thio)cyanate, butyl iso(thio)cyanate, propyl iso(thio)cyanate, ethyl iso(thio)cyanate, octadecyl iso(thio)cyanate, heptyl iso(thio)cyanate, isopropyl iso(thio)cyanate, octyl iso(thio)cyanate, tert-butyl iso(thio)cyanate, and benzyl iso(thio)cyanate.

Examples of di- or higher functional iso(thio)cyanate compounds include aliphatic polyisocyanate compounds, alicyclic polyisocyanate compounds, aromatic polyisocyanate compounds, heterocyclic polyisocyanate compounds, aliphatic polyisothiocyanate compounds, alicyclic polyisothiocyanate compounds, aromatic polyisothiocyanate compounds, sulfur-containing heterocyclic polyisothiocyanate compounds, and modified products thereof.

The iso(thio)cyanate compound in the first embodiment preferably contains a bifunctional isocyanate compound from the viewpoint of the handleability of the urethane (meth)acrylate to be obtained.

More specific examples of di- or higher-functional isocyanate compounds include aliphatic polyisocyanate compounds such as pentamethylene diisocyanate, hexamethylene diisocyanate, 2,2,4-trimethylhexane diisocyanate, 2,2,4-trimethylhexamethylene diisocyanate, 2,4,4-trimethylhexamethylene diisocyanate, lysine diisocyanatomethyl ester, lysine triisocyanate, m-xylylene diisocyanate, p-xylylene diisocyanate, 1,3-tetramethylxylylene diisocyanate, $\alpha,\alpha,\alpha',\alpha'$-tetramethylxylylene diisocyanate, bis(isocyanatomethyl)naphthaline, mesitylene triisocyanate, bis(isocyanatomethyl)sulfide, bis(isocyanatoethyl)sulfide, bis(isocyanatomethyl)disulfide, bis(isocyanatoethyl)disulfide, bis(isocyanatomethylthio)methane, bis(isocyanatoethylthio)methane, bis(isocyanatoethylthio)ethane, and bis(isocyanatomethylthio)ethane;

alicyclic polyisocyanate compounds such as isophorone diisocyanate, bis(isocyanatomethyl)cyclohexane, dicyclohexylmethane-4,4'-diisocyanate, cyclohexane diisocyanate, methylcyclohexane diisocyanate, dicyclohexyldimethylmethane isocyanate, 2,5-bis(isocyanatomethyl)bicyclo-[2.2.1]-heptane, 2,6-bis(isocyanatomethyl)bicyclo-[2.2.1]-heptane, 3,8-bis(isocyanatomethyl)tricyclodecane, 3,9-bis(isocyanatomethyl)tricyclodecane, 4,8-bis(isocyanatomethyl)tricyclodecane, and 4,9-bis(isocyanatomethyl)tricyclodecane;
aromatic polyisocyanate compounds such as phenylene diisocyanate, 2,4-tolylene diisocyanate, 2,6-tolylene diisocyanate, 4,4'-diphenylmethane diisocyanate, and diphenyl sulfide-4,4-diisocyanate; and
heterocyclic polyisocyanate compounds such as 2,5-diisocyanatothiophene, 2,5-bis(isocyanatomethyl)thiophene, 2,5-diisocyanatotetrahydrothiophene, 2,5-bis(isocyanatomethyl)tetrahydrothiophene, 3,4-bis(isocyanatomethyl)tetrahydrothiophene, 2,5-diisocyanato-1,4-dithiane, 2,5-bis(isocyanatomethyl)-1,4-dithiane, 4,5-diisocyanato-1,3-dithiolane, and 4,5-bis(isocyanatomethyl)-1,3-dithiolane.

In addition, a halogen-substituted product such as a chlorine-substituted product, and a bromine-substituted product, an alkyl-substituted product, an alkoxy-substituted product, a nitro-substituted product, a prepolymer-type product modified with a polyhydric alcohol, a carbodiimide-modified product, a urea-modified product, a biuret-modified product, a dimerization or trimerization reaction product, and the like can also be used.

Among the bi- or higher functional isocyanate compounds, it is preferable to include at least one selected from the group consisting of a mixture of 2,2,4-trimethylhexamethylene diisocyanate and 2,4,4-trimethylhexamethylene diisocyanate, isophorone diisocyanate, m-xylylene diisocyanate, 1,3-tetramethylxylylene diisocyanate, a mixture of 2,5-bis(isocyanatomethyl)bicyclo-[2.2.1]-heptane and 2,6-bis(isocyanatomethyl)bicyclo-[2.2.1]-heptane, and $\alpha,\alpha,\alpha',\alpha'$-tetramethylxylylene diisocyanate.

<Hydroxy (Meth)acrylate Compound>

The hydroxy (meth)acrylate compound in the first embodiment contains a hydroxy group and a (meth)acryloyloxy group.

The hydroxy (meth)acrylate compound in the first embodiment is a (meth)acrylate compound other than the urethane (meth)acrylate in the first embodiment.

Examples of the hydroxy (meth)acrylate compound include 2-hydroxyethyl (meth)acrylate, 2-hydroxypropyl (meth)acrylate, 2-hydroxybutyl (meth)acrylate, 2-hydroxy-3-phenoxypropyl (meth)acrylate, 4-hydroxybutyl (meth)acrylate, and 1,4-cyclohexanedimethanol mono(meth)acrylate.

The hydroxy (meth)acrylate compound in the first embodiment preferably includes, among the above, at least one selected from the group consisting of 2-hydroxyethyl (meth)acrylate, 2-hydroxypropyl (meth)acrylate, 2-hydroxybutyl (meth)acrylate, 2-hydroxy-3 phenoxypropyl (meth)acrylate, 4-hydroxybutyl (meth)acrylate, and 1,4-cyclohexanedimethanol mono(meth)acrylate, and more preferably contains 2-hydroxyethyl acrylate.

The urethane (meth)acrylate preferably contains a compound represented by the following Formula (1).

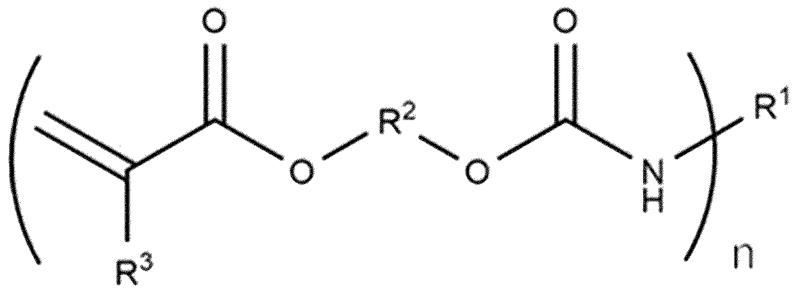

Formula (1)

In Formula (1), $R^1$ is a monovalent organic group or a divalent linking group, $R^2$ is each independently a divalent linking group, $R^3$ is each independently a methyl group or a hydrogen atom, and n is 1 or 2.

In Formula (1), $R^1$ is a monovalent organic group when n is 1, and is a divalent linking group when n is 2.

In the above Formula (1), when n is 2, $R_1$ is preferably a divalent organic group, and more preferably a divalent organic group which may contain at least one selected from the group consisting of an aromatic structure, an alicyclic structure, an ether bond, an ester bond, and a urethane bond.

In $R^1$ of Formula (1), when n is 2, the divalent organic group preferably contains a divalent chain hydrocarbon group. The divalent organic group may be a divalent chain hydrocarbon group.

In Formula (1), when n is 1, $R^1$ is preferably a monovalent organic group which may contain at least one selected from the group consisting of an aromatic structure, an alicyclic structure, an ether bond, an ester bond, and a urethane bond.

In $R^1$ of the Formula (1), when n is 1, the monovalent organic group preferably contains a monovalent chain hydrocarbon group. The monovalent organic group may be a monovalent chain hydrocarbon group.

$R^1$ in Formula (1) may be a divalent chain hydrocarbon group or a monovalent chain hydrocarbon group.

The divalent chain hydrocarbon group or the monovalent chain hydrocarbon group may be saturated or unsaturated, and may have a substituent. The divalent chain hydrocarbon group may be a linear or branched chain alkylene group.

The monovalent chain hydrocarbon group may be a linear or branched chain alkyl group.

In $R^1$ of Formula (1), the number of carbon atoms of the divalent organic group or the number of carbon atoms of the monovalent organic group may be, for example, in the range of from 2 to 250, and is preferably in the range of from 3 to 100.

In $R^1$ of Formula (1), the number of carbon atoms of the divalent organic group or the monovalent organic group may contain a heteroatom. Examples of the heteroatom include an oxygen atom and a nitrogen atom.

In $R^1$ of Formula (1), when n is 2, examples of the divalent hydrocarbon group having an aromatic structure can include an arylene group, an alkylene arylene group, an alkylene arylene alkylene group, and an arylene alkylene arylene group.

In $R^1$ of Formula (1), when n is 2, examples of the divalent hydrocarbon group having an alicyclic structure can include a cyclopropylene group, a cyclobutylene group, a cyclopentylene group, a cyclohexylene group, a cyclohexenylene group, a cycloheptylene group, a cyclooctylene group, a cyclononylene group, a cyclodecylene group, a cycloundecylene group, a cyclododecylene group, a cyclotridecylene group, a cyclotetradecylene group, a cyclopentadecylene group, a cyclooctadecylene group, a cycloicosylene group, a bicyclohexylene group, a norbornylene group, an isobornylene group, and an adamantylene group.

In $R^1$ of Formula (1), when n is 1, examples of the monovalent hydrocarbon group having an aromatic structure include an aryl group, an alkylene aryl group, an alkylene arylene alkyl group, arylene alkylene aryl group.

In $R^1$ of Formula (1), when n is 1, examples of the monovalent hydrocarbon group having an alicyclic structure can include a cyclopropyl group, a cyclobutyl group, a cyclopentyl group, a cyclohexyl group, a cyclohexenyl group, a cycloheptyl group, a cyclooctyl group, a cyclononyl group, a cyclodecyl group, a cycloundecyl group, a cyclododecyl group, a cyclotridecyl group, a cyclotetradecyl group, a cyclopentadecyl group, a cyclooctadecyl group, a cycloicosyl group, a bicyclohexyl group, a norbornyl group, an isobornyl group, and an adamantyl group.

$R^1$ in Formula (1) may have a substituent, and examples of the substituent can include a linear or branched alkyl group having from 1 to 6 carbon atoms.

In Formula (1), it is preferable that $R^2$ is each independently a divalent chain hydrocarbon group which may contain a substituent.

The divalent chain hydrocarbon group suitable as $R^2$ is the same as the divalent chain hydrocarbon group suitable as $R^1$. However, the divalent chain hydrocarbon group as $R^2$ preferably has from 2 to 6 carbon atoms, and more preferably has from 2 to 3 carbon atoms. In addition, the divalent chain hydrocarbon group as $R^2$ is preferably a divalent chain hydrocarbon group having from 2 to 6 carbon atoms and having no substituent, more preferably from 2 to 3 carbon

atoms, and still more preferably 2 carbon atoms, from the viewpoint of being capable of suppressing the viscosity.

When $R^2$ has a substituent, examples of the substituent include an alkyl group having from 1 to 6 carbon atoms such as a methyl group and an ethyl group; an aryl group; a cycloalkyl groups having from 3 to 6 carbon atoms, such as a cyclopentyl group and a cyclohexyl group; a tolyl group; a xylyl group; a cumyl group; a styryl group; and an alkoxyphenyl group such as a methoxyphenyl group, an ethoxyphenyl group, and a propoxyphenyl group.

$R^3$ is preferably a hydrogen atom.

As the molecular weight of the urethane (meth)acrylate, the weight average molecular weight is preferably from 150 to 10,000, more preferably from 200 to 5,000, and still more preferably 250 to 3,000.

<2-hydroxyethyl Acrylate>

The urethane (meth)acrylate composition of the first embodiment contains 2-hydroxyethyl acrylate.

The urethane (meth)acrylate composition of the first embodiment contains 2-hydroxyethyl acrylate, the content of 2-hydroxyethyl acrylate is 10,000 ppm by mass or less, and accordingly, a stereolithography product having excellent antibacterial properties and biocompatibility can be obtained.

The content of 2-hydroxyethyl acrylate is 10,000 ppm by mass or less with respect to the total mass of the urethane (meth)acrylate composition, and accordingly, excellent biocompatibility is obtained.

From the above viewpoint, the content of 2-hydroxyethyl acrylate is preferably 7,000 ppm by mass or less, more preferably 4,000 ppm by mass or less, still more preferably 3,500 ppm by mass or less, particularly preferably 3,000 ppm by mass or less, and further more preferably 2,000 ppm by mass or less, with respect to the total mass of the urethane (meth)acrylate composition.

The content of 2-hydroxyethyl acrylate is preferably 5 ppm by mass or more with respect to the total mass of the composition.

The content of 2-hydroxyethyl acrylate is 5 ppm by mass or more with respect to the total mass of the composition, and accordingly, excellent antibacterial properties are obtained.

From the above viewpoint, the content of 2-hydroxyethyl acrylate is more preferably 10 ppm by mass or more, still more preferably 15 ppm by mass or more, and particularly preferably 20 ppm by mass or more, with respect to the total mass of the composition.

The composition of the first embodiment may contain other components such as a photopolymerization initiator, but it is also preferable that, in a case in which the composition of the first embodiment does not contain other components, the content of 2-hydroxyethyl acrylate preferably satisfies the above range.

«Photocurable Composition»

The composition of the first embodiment preferably further contains a photopolymerization initiator.

Hereinafter, among the compositions of the first embodiment, a composition containing a photopolymerization initiator may be referred to as a "photocurable composition".

The photocurable composition of the first embodiment may contain a (meth)acrylic monomer (C) (hereinafter also referred to as a (meth)acrylic monomer (C)) other than urethane (meth)acrylate and 2-hydroxyethyl acrylate.

The (meth)acrylic monomer (C) can be used without particular limitation, and a known (meth)acrylic monomer may be used. Examples of the (meth)acrylic monomer include monofunctional (meth)acrylate, bifunctional (meth)acrylate, and tri- or higher functional (meth)acrylate.

Examples of the monofunctional (meth)acrylate include monofunctional (meth)acrylates containing no urethane bond.

Examples of the monofunctional (meth)acrylate not containing a urethane bond include cyclohexyl (meth)acrylate, isobornyl (meth)acrylate, dicyclopentanyl (meth)acrylate, dicyclopentenyl (meth)acrylate, 4-tert-butylcyclohexyl (meth)acrylate, tetrahydrofurfuryl (meth)acrylate, (2-methyl-2-ethyl-1,3-dioxolane-4-yl)methyl (meth)acrylate, cyclic trimethylolpropane formal (meth)acrylate, 4-(meth)acryloylmorpholine, lauryl (meth)acrylate, 4-hydroxybutyl (meth)acrylate, 2-hydroxyethyl (meth)acrylate, benzyl (meth)acrylate, 2-phenoxyethyl (meth)acrylate, 3-phenoxybenzyl (meth)acrylate, 2-hydroxy-3-phenoxypropyl (meth)acrylate, phenoxyethylene glycol (meth)acrylate, 2-dodecyl-1-hexadecanyl (meth)acrylate, 2-(meth)acryloyloxyethyl-succinic acid, 2-[[(butylamino)carbonyl]oxy]ethyl (meth)acrylate, 2-(2-ethoxyethoxy)ethyl (meth)acrylate, and ethoxylated-o-phenylphenol acrylate.

Examples of the bifunctional (meth)acrylate include bifunctional (meth)acrylates containing no urethane bond.

Examples of the bifunctional (meth)acrylate not containing a urethane bond include ethylene glycol di(meth)acrylate, polyethylene glycol di(meth)acrylate (triethylene glycol di(meth)acrylate and the like), polypropylene glycol di(meth)acrylate, glycerin di(meth)acrylate, 1,6-hexanediol di(meth)acrylate, dimethylol-tricyclodecane di(meth)acrylate, 1,9-nonanediol di(meth)acrylate, dioxane glycol di(meth)acrylate, diethylene glycol di(meth)acrylate, dipropylene glycol di(meth)acrylate, ethoxylated bisphenol A di(meth)acrylate, and ethoxylated hydrogenated bisphenol A di(meth)acrylate.

Examples of the tri- or higher (meth)acrylate include tri- or higher (meth)acrylates not containing a urethane bond.

Examples of the tri- or higher (meth)acrylate not containing a urethane bond include trimethylolpropane tri(meth)acrylate, tetramethylolmethane tetra(meth)acrylate, ethoxylated trimethylolpropane triacrylate (EO = 3 mol), propoxylated trimethylolpropane triacrylate (PO = 3 mol), propoxylated glycerin triacrylate (PO = 3 mol), and caprolactone-modified tris-(2-acryloxyethyl) isocyanurate triacrylate.

In the photocurable composition of the first embodiment, the content ratio of the urethane (meth)acrylate and the (meth)acrylic monomer (C) is preferably from 90: 10 to 10:90, more preferably from 80:20 to 20:80, and still more preferably from 70:30 to 30:70.

In the photocurable composition of the first embodiment, the content of the bifunctional (meth)acrylate and the monofunctional (meth)acrylate is preferably 80% by mass or more, more preferably 90% by mass or more, and still more preferably 95% by mass or more, with respect to the total mass of the (meth)acrylate in the photocurable composition.

Examples of the monofunctional (meth)acrylate include a monofunctional urethane (meth)acrylate among the urethane (meth)acrylates, and a monofunctional (meth)acrylate not containing a urethane bond among the (meth)acrylic monomers (C).

Examples of the bifunctional (meth)acrylate include a bifunctional urethane (meth)acrylate among the urethane (meth)acrylates, and a bifunctional (meth)acrylate not containing a urethane bond among the (meth)acrylic monomers (C).

The photopolymerization initiator is not particularly limited as long as the photopolymerization initiator generates radicals by irradiating light, but the photopolymerization initiator preferably generates radicals at the wavelength of light used in stereolithography.

The wavelength of light used in stereolithography is generally from 365 nm to 500 nm, but practically preferably from 365 nm to 430 nm, and more preferably from 365 nm to 420 nm.

Examples of the photopolymerization initiator that generates radicals at a wavelength of light used in stereolithography include alkylphenone compounds, acylphosphine oxide compounds, titanocene compounds, oxime ester compounds, benzoin compounds, acetophenone compounds, benzophenone compounds, thioxanthone compounds, $\alpha$-acyloxime ester compounds, phenylglyoxylate compounds, benzyl compounds, azo compounds, diphenyl sulfide compounds, organic dye compounds, iron-phthalocyanine compounds, benzoin ether compounds, and anthraquinone compounds.

Among these compounds, an alkylphenone compound and an acylphosphine oxide compound are preferable from the viewpoint of reactivity and the like.

Examples of the alkylphenone compound include 1-hydroxy-cyclohexyl-phenyl-ketone (Omnirad 184: manufactured by IGM Resins B.V).

Examples of the acylphosphine oxide compound include bis(2,4,6-trimethylbenzoyl)-phenylphosphine oxide (Omnirad 819: manufactured by IGM Resins B.V) and 2,4,6-trimethylbenzoyl-diphenyl-phosphine oxide (Omnirad TPO: manufactured by IGM Resins B.V).

The photocurable composition of the first embodiment may contain only one kind or two or more kinds of photopolymerization initiators.

The content (the total content in the case of two or more kinds) of the photopolymerization initiator in the photocurable composition of the first embodiment is preferably from 0.1% by mass to 10% by mass, more preferably from 0.2% by mass to 5% by mass, and still more preferably from 0.3% by mass to 3% by mass, with respect to the total mass of the photocurable composition.

In the photocurable composition of the first embodiment, the content of the urethane (meth)acrylate is preferably 10% by mass or more, more preferably 20% by mass or more, still more preferably 25% by mass or more, and particularly preferably 30% by mass or more, with respect to the total mass of the photocurable composition.

The content of the urethane (meth)acrylate may be 95% by mass or less, 90% by mass or less, or 80% by mass or less with respect to the total mass of the photocurable composition.

In the photocurable composition of the first embodiment, the content of the urethane (meth)acrylate may be 5% by mass or more and 98% by mass or less with respect to the total mass of the photocurable composition.

In the photocurable composition in the first embodiment, the cured product of the photocurable composition preferably has an IC50 of 40% or more.

In the first embodiment, a cured product of a photocurable composition for measuring the IC50 is obtained by the following method.

The photocurable composition is irradiated with visible light having a wavelength of 405 nm at an irradiation amount of 12 $mJ/cm^2$ to form a cured layer P1 having a thickness of 100 $\mu$m. The cured layer P1 is layered in the thickness direction to form the molded article P1 having a rectangular parallelepiped shape with a length of 20 mm, a width of 20 mm, and a thickness of 2 mm. The obtained molded article P1 is immersed in isopropanol and washed for 5 minutes using an ultrasonic washer having an output of 60 W. After the washed molded article P1 is dried by air blowing, by stereolithography under the condition of irradiating the molded article P1 with ultraviolet light having a wavelength of 365 nm at an irradiation amount of 10 $J/cm^2$, a test piece P1 having a rectangular parallelepiped shape

with a length of 20 mm, a width of 20 mm, and a thickness of 2 mm is obtained. The obtained test piece P1 is a cured product of a photocurable composition for measuring the IC50.

The measurement of IC50 of the cured product of the photocurable composition in the first embodiment is performed according to ISO 10993-5: 2009 Annex B.

<Viscosity>

The photocurable composition of the first embodiment preferably has a viscosity measured under conditions of 25°C and 50 rpm by an E-type viscometer (hereinafter also simply referred to as "viscosity") of from 5 mPa s to 20,000 mPa·s.

Here, rpm means revolutions per minute (that is, rotations per minute).

When the viscosity is from 5 mPa·s to 20,000 mPa·s, the handleability of the photocurable composition when manufacturing a stereolithography product by stereolithography is excellent.

The viscosity is more preferably from 10 mPa s to 6,000 mPa·s, still more preferably from 20 mPa·s to 5,000 mPa·s, particularly preferably from 100 mPa·s to 4,000 mPa·s, further more preferably from 200 mPa·s to 3,000 mPa·s, and even more preferably 400 mPa·s to 2,000 mPa·s.

<Other Additives>

The photocurable composition of the first embodiment may contain one or more additives other than the above-described components as necessary.

In a case in which the photocurable composition contains another additive, the total mass of the urethane (meth)acrylate, 2-hydroxyethyl acrylate, the (meth)acrylic monomer (C), and the photopolymerization initiator is preferably 30% by mass or more, more preferably 50% by mass or more, still more preferably 70% by mass or more, particularly preferably 80% by mass or more, and further more preferably 90% by mass or more, with respect to the total amount of the photocurable composition.

Examples of the other component include an additive such as a filler, a coloring material, a coupling agent such as a silane coupling agent (for example, 3-acryloxypropyltrimethoxysilane), a rubber agent, an ion trapping agent, an ion exchange agent, a leveling agent, a plasticizer, and an antifoaming agent.

The method for preparing the photocurable composition of the first embodiment is not particularly limited, and examples thereof include a method of mixing the respective components.

Means for mixing the respective components is not particularly limited, and examples thereof include means such as dissolution by ultrasonic waves, a double arm stirrer, a roll kneader, a twin screw extruder, a ball mill kneader, and a planetary stirrer.

The photocurable composition of the present embodiment may be prepared by mixing the respective components, filtering the mixture with a filter to remove impurities, and further subjecting the mixture to a vacuum defoaming treatment.

<<Urethane (Meth)acrylate Composition>>

In the composition of the first embodiment, the content of the urethane (meth)acrylate may be 90% by mass or more with respect to the total mass of the photocurable composition.

Hereinafter, in the first embodiment, a composition in which the content of the urethane (meth)acrylate is 90% by mass or more with respect to the total mass of the composition is also referred to as a "urethane (meth)acrylate composition".

In the urethane (meth)acrylate composition in the first embodiment, the content of the urethane (meth)acrylate is preferably 95% by mass or more, and more preferably 97% by mass or more with respect to the total mass of the urethane (meth)acrylate composition.

The content of the urethane (meth)acrylate may be 99% by mass or less with respect to the total mass of the urethane (meth)acrylate composition.

The urethane (meth)acrylate composition of the first embodiment may contain other components such as a photopolymerization initiator.

<<Cured Product>>

The method for performing photocuring using the photocurable composition of the first embodiment is not particularly limited, and any of known methods and apparatuses can be used. Examples thereof include a method of layering a plurality of cured layers by repeating a step of forming a thin film made of the photocurable composition of

the first embodiment and a step of irradiating the thin film with light to obtain a cured layer a plurality of times to manufacture a cured product having a desired shape. The resulting cured product may be used as it is, or may be used after being further subjected to post curing by light irradiation, heating, or the like to improve the mechanical properties, shape stability, and the like thereof.

< Stereolithography >

The photocurable composition of the first embodiment is preferably used for stereolithography.
Among them, the photocurable composition of the first embodiment can be suitably used for a molding method using a 3D printer.
In the first embodiment, "stereolithography" is one type of three-dimensional molding method using a 3D printer.
The stereolithography may be inkjet type stereolithography or liquid tank type stereolithography (that is, stereolithography using a liquid tank).
From the viewpoint of more effectively exhibiting the effect of the photocurable composition of the first embodiment, the stereolithography is preferably a liquid tank type stereolithography.
Examples of the liquid tank type stereolithography include digital light processing (DLP) type stereolithography, liquid crystal display (LCD) type stereolithography, and stereolithography (SLA) type stereolithography.
In the DLP type and the LCD type, the photocurable composition in the liquid tank is irradiated with planar light.
In the SLA type, the photocurable composition in the liquid tank is scanned with a laser light.
From the viewpoint of more effectively exhibiting the effect of the photocurable composition of the first embodiment, the liquid tank type stereolithography is preferably a DLP type stereolithography.

< Stereolithography Product >

The stereolithography product of the first embodiment is a stereolithography product of the photocurable composition of the first embodiment.
The stereolithography product of the first embodiment is excellent in antibacterial properties and biocompatibility.

(IC50)

In the stereolithography product of the first embodiment, the IC50 is preferably 5% or more.
It is considered that the higher the value of IC50 is, the more excellent biocompatibility is exhibited.
Since the stereolithography product of the first embodiment is the stereolithography product of the photocurable composition of the first embodiment, the value of the IC50 can be improved. Therefore, biocompatibility is excellent.
From the above viewpoint, in the stereolithography product of the first embodiment, the IC50 is more preferably 10% or more, still more preferably 30% or more, particularly preferably 40% or more, even more preferably 50% or more, further more preferably 60% or more, and even more preferably 70% or more.
The value of IC50 is measured by the method of ISO 10993-5: 2009 Annex B.

<3D Printer>

Examples of the stereolithography type include a stereolithography apparatus (SLA) type, a digital light processing (DLP) type, a liquid crystal display (LCD) type, and an inkjet type.
The photocurable composition of the present embodiment is particularly suitable for stereolithography of an SLA type, a DLP type, or an LCD type.
Examples of the SLA type include a type of irradiating the photocurable composition with spot-shaped ultraviolet laser light to obtain a three-dimensional object.
In the case of preparing a dental product or the like by the SLA method, for example, the photocurable composition of the present embodiment may be stored in a container, the photocurable composition may be cured by selectively irradiating a liquid surface of the photocurable composition with spot-shaped ultraviolet laser light to obtain a desired pattern, a cured layer having a desired thickness may be formed on the molding table, then the molding table may be lowered, one layer of the liquid photocurable composition may be supplied onto the cured layer, and the liquid photocurable composition may be similarly cured to repeat the layering operation to obtain a continuous cured layer. Thus, a dental product or the like can be prepared.
Examples of the DLP type include a type of irradiating the photocurable composition with planar light to obtain a three-dimensional object.
For a method of obtaining a three-dimensional object by the DLP type, for example, the description of Japanese Patent No. 5111880 and Japanese Patent No. 5235056 can be appropriately referred to.

When a dental product or the like is prepared by the DLP type, for example, a lamp that emits light other than laser light, such as a high-pressure mercury lamp, an ultra-high-pressure mercury lamp, or a low-pressure mercury lamp, an LED, or the like is used as a light source, a planar drawing mask having a plurality of digital micromirror shutters arranged in a planar shape is disposed between the light source and the modeled surface of the photocurable composition, and light is irradiated to the modeled surface of the photocurable composition through the planar drawing mask to sequentially layer the cured layers having a predetermined shape pattern. Thus, a dental product or the like can be prepared.

Examples of the LCD type include a type of irradiating the photocurable composition with planar light to obtain a three-dimensional object.

When a dental product or the like is prepared by the LCD type, for example, a lamp (for example, a high-pressure mercury lamp, an ultra-high-pressure mercury lamp, a low-pressure mercury lamp, or the like) that emits light other than laser light, an LED, or the like can be used as the light source.

When a dental product or the like is prepared by an LCD type, a liquid crystal display may be disposed between a light source and a modeled surface of a photocurable composition, and the modeled surface of the photocurable composition may be irradiated with light through the liquid crystal display to sequentially layer the cured layers having a predetermined shape pattern. Thus, a dental product or the like can be prepared.

Examples of the inkjet type include a type in which droplets of a photocurable composition are continuously discharged from an inkjet nozzle to a base material, and the droplets attached to the base material are irradiated with light to obtain a three-dimensional obj ect.

When a dental product or the like is prepared by an inkjet type, for example, a photocurable composition is discharged from an inkjet nozzle to a base material while a head including the inkjet nozzle and a light source is scanned in a plane, the discharged photocurable composition is irradiated with light to form a cured layer, and these operations are repeated to sequentially layer the cured layers. Thus, a dental product or the like can be prepared.

<<Dental Product>>

The dental product of the first embodiment includes the stereolithography product of the first embodiment.

The photocurable composition of the first embodiment is preferably used for manufacturing a dental product.

The photocurable composition of the first embodiment is more preferably used for manufacturing a dental product by stereolithography.

The dental product of the first embodiment preferably contains a cured product of the photocurable composition of the first embodiment.

The dental product containing the cured product (that is, a stereolithography product) of the photocurable composition of the first embodiment is not particularly limited, and can be used in an artificial tooth, a prosthesis, a medical instrument used in the oral cavity, a model (zinc diver mask or the like), and the like, but a prosthesis or an instrument used in the oral cavity is preferable. The cured product of the photocurable composition of the first embodiment is preferably used for at least a part of a dental product.

Examples of the prosthesis can include a complete denture and a partial denture. Examples of the instrument used in the oral cavity can include a sports mouthpiece, a mouthguard, a mouthpiece for orthodontics, a splint such as a splint for occlusal adjustment or a splint for treatment of temporomandibular joint disorder, and a mouthpiece used for treatment of sleep apnea syndrome.

By using the cured product of the photocurable composition of the first embodiment, a medical device having antibacterial properties and biocompatibility can be manufactured.

The first embodiment also includes the following aspects.

<1-1> A composition containing urethane (meth)acrylate and 2-hydroxyethyl acrylate, in which the content of the 2-hydroxyethyl acrylate is 4,000 ppm by mass or less with respect to the total mass of the composition.

<1-2> The composition according to <1-1>, in which the content of the 2-hydroxyethyl acrylate is 5 ppm by mass or more with respect to the total mass of the composition.

<1-3> The composition according to <1-1> or <1-2>, in which the urethane (meth)acrylate contains a urethane (meth)acrylate having 2 or less functional groups.

<1-4> The composition according to any one of <1-1> to <1-3>, further containing a photopolymerization initiator.

<1-5> The composition according to <1-4>, further containing a (meth)acrylic monomer (C) other than the urethane (meth)acrylate and the 2-hydroxyethyl acrylate.

<1-6> The composition according to <1-4> or <1-5>, in which IC50 of the cured product is 40% or more.

<1-7> The composition according to any one of <1-4> to <1-6>, in which the content of the bifunctional (meth)acrylate and the monofunctional (meth)acrylate is 80% by mass or more with respect to the total mass of the (meth)acrylate in the composition.

<1-8> The composition according to any one of <1-4> to <1-7>, in which the content of the urethane (meth)acrylate is 25% by mass or more with respect to the total mass of the composition.
<1-9> The composition according to any one of <1-1> to <1-3>, in which the content of the urethane (meth)acrylate is 95% by mass or more with respect to the total mass of the composition.
<1-10> The composition according to any one of <1-4> to <1-8>, which is used for stereolithography.
<1-11> The composition according to any one of <1-1> to <1-10>, which is used for manufacturing a dental product.
<1-12> A stereolithography product of the composition according to any one of <1-4> to <1-8>.
<1-13> The stereolithography product according to <1-12>, in which the IC50 is 40% or more.
<1-14> A dental product including the stereolithography product according to <1-12> or <1-13>.

[Second Embodiment]

<<Composition>>

The composition of the second embodiment contains urethane (meth)acrylate and hydroxypropyl acrylate, and the content of the hydroxypropyl acrylate is 25,000 ppm by mass or less with respect to the total mass of the composition.
The composition of the second embodiment contains a combination of urethane (meth)acrylate and hydroxypropyl acrylate, the content of hydroxypropyl acrylate is within the above range, and accordingly, a stereolithography product having excellent antibacterial properties and biocompatibility can be obtained.
The composition of the second embodiment may be composed of only urethane (meth)acrylate and hydroxypropyl acrylate, or may further contain components other than urethane (meth)acrylate and hydroxypropyl acrylate.

<Urethane (Meth)acrylate>

The composition of the second embodiment contains a urethane (meth)acrylate.
The details of specific aspects, preferable aspects, definitions, and the like of the urethane (meth)acrylate in the second embodiment are the same as the details of specific aspects, preferable aspects, definitions, and the like of the urethane (meth)acrylate in the first embodiment.
For example, similar to the first embodiment, the urethane (meth)acrylate in the second embodiment may be a reaction product of an iso(thio)cyanate compound and a hydroxy (meth)acrylate compound containing a hydroxy group and a (meth)acryloyloxy group. In addition, for example, the urethane (meth)acrylate in the second embodiment may be a reaction product of an isocyanate compound, a hydroxy (meth)acrylate compound containing a hydroxy group and a (meth)acryloyloxy group, and a compound other than these compounds (for example, a thiol compound containing a mercapto group).

<Iso(thio)cyanate Compound>

Examples of the iso(thio)cyanate compound include a monofunctional iso(thio)cyanate compound and a di- or higher functional iso(thio)cyanate compound.
The iso(thio)cyanate compounds may be used singly or in combination of two or more kinds thereof.
The details of specific examples, preferable specific examples, preferable aspects, and the like of the monofunctional iso(thio)cyanate compound and the di- or higher functional iso(thio)cyanate compound in the second embodiment are the same as the details of specific examples, preferable specific examples, preferable aspects, and the like of the monofunctional iso(thio)cyanate compound and the di- or higher functional iso(thio)cyanate compound in the first embodiment.

<Hydroxy (Meth)acrylate Compound>

The hydroxy (meth)acrylate compound in the second embodiment contains a hydroxy group and a (meth)acryloyloxy group.
The hydroxy (meth)acrylate compound in the second embodiment is a (meth)acrylate compound other than the urethane (meth)acrylate in the second embodiment.
The details of specific examples, preferable specific examples, preferable aspects, and the like of the hydroxy (meth)acrylate compound in the second embodiment are the same as the details of specific examples, preferable specific examples, preferable aspects, and the like of the hydroxy (meth)acrylate compound in the first embodiment. The preferable specific examples include, of course, the compound represented by Formula (1), and also include a preferable aspect of the compound represented by Formula (1).

<Hydroxypropyl Acrylate>

The urethane (meth)acrylate composition of the second embodiment contains hydroxypropyl acrylate.

The urethane (meth)acrylate composition of the second embodiment contains hydroxypropyl acrylate, the content of hydroxypropyl acrylate is 25,000 ppm by mass or less, and accordingly, a stereolithography product having excellent antibacterial properties and biocompatibility can be obtained.

There are three isomers of "hydroxypropyl acrylate": 2-hydroxypropyl acrylate, 2-hydroxy-1-methylethyl acrylate, and 3-hydroxypropyl acrylate.

The "hydroxypropyl acrylate" in the second embodiment means a hydroxypropyl acrylate containing at least one selected from the group consisting of 2-hydroxypropyl acrylate, 2-hydroxy-1-methylethyl acrylate, and 3-hydroxypropyl acrylate.

The "hydroxypropyl acrylate" in the second embodiment is a concept also including a mixture of two or more selected from the group consisting of 2-hydroxypropyl acrylate, 2-hydroxy-1-methylethyl acrylate, and 3-hydroxypropyl acrylate.

The hydroxypropyl acrylate in the second embodiment preferably contains at least one of 2-hydroxypropyl acrylate or 2-hydroxy-1-methylethyl acrylate, and more preferably contains a mixture of 2-hydroxypropyl acrylate and 2-hydroxy-1-methylethyl acrylate.

The content of hydroxypropyl acrylate is 25,000 ppm by mass or less with respect to the total mass of the urethane (meth)acrylate composition, and accordingly, biocompatibility is excellent.

From the above viewpoint, the content of hydroxypropyl acrylate is preferably 20,000 ppm by mass or less, and more preferably 15,000 ppm by mass or less, with respect to the total mass of the urethane (meth)acrylate composition.

The content of hydroxypropyl acrylate is preferably 10 ppm by mass or more with respect to the total mass of the composition.

The content of hydroxypropyl acrylate is 10 ppm by mass or more with respect to the total mass of the composition, and accordingly, excellent antibacterial properties are obtained.

From the above viewpoint, the content of hydroxypropyl acrylate is more preferably 20 ppm by mass or more, still more preferably 30 ppm by mass or more, and particularly preferably 40 ppm by mass or more, with respect to the total mass of the composition.

The composition of the second embodiment may contain other components such as a photopolymerization initiator, but in a case in which the composition of the second embodiment does not contain other components, it is also preferable that the content of hydroxypropyl acrylate preferably satisfies the above range.

The content of hydroxypropyl acrylate is preferably from 10 ppm by mass to 25,000 ppm by mass, and more preferably from 20 ppm by mass to 20,000 ppm by mass, with respect to the total mass of the composition.

«Photocurable Composition»

The composition of the second embodiment preferably further contains a photopolymerization initiator.

Hereinafter, among the compositions of the second embodiment, a composition containing a photopolymerization initiator may be referred to as a "photocurable composition".

The photocurable composition of the second embodiment may contain a (meth)acrylic monomer (D) (hereinafter also referred to as a (meth)acrylic monomer (D)) other than urethane (meth)acrylate and hydroxypropyl acrylate.

The details of specific examples, preferable specific examples, preferable aspects and the like of the (meth)acrylic monomer (D) in the second embodiment are the same as the details of specific examples, preferable specific examples, preferable aspects and the like corresponding to (meth)acrylic monomers other than urethane (meth)acrylate and hydroxypropyl acrylate in the description of the (meth)acrylic monomer (C) in the first embodiment.

The details of specific examples, preferable specific examples, preferable aspects, preferable contents, and the like of the photopolymerization initiator in the second embodiment are the same as the details of specific examples, preferable specific examples, preferable aspects, preferable contents, and the like of the photopolymerization initiator in the first embodiment.

The preferable range of the content of the urethane (meth)acrylate in the second embodiment is the same as the preferable range of the content of the urethane (meth)acrylate in the first embodiment.

In the photocurable composition in the second embodiment, the cured product of the photocurable composition preferably has an IC50 of 5% or more.

In the second embodiment, a cured product of a photocurable composition for measuring the IC50 is obtained by the method described in the first embodiment.

The IC50 of the cured product of the photocurable composition in the second embodiment is measured by the same method as the method described in the first embodiment.

<Viscosity>

The preferable range of the viscosity in the photocurable composition of the second embodiment is the same as the preferable range of the viscosity in the photocurable composition of the first embodiment.

<Other Additives>

The photocurable composition of the second embodiment may contain one or more additives other than the above-described components as necessary.

The details of the preferable content, specific examples, and the like of the additive in the second embodiment are the same as the details of the preferable content, specific examples, and the like of the additive in the first embodiment.

The specific aspect of the method for preparing a photocurable composition of the second embodiment is the same as the specific aspect of the method for preparing a photocurable composition of the first embodiment.

<<Urethane (Meth)acrylate Composition>>

In the composition of the second embodiment, the content of the urethane (meth)acrylate may be 90% by mass or more with respect to the total mass of the photocurable composition.

Hereinafter, in the second embodiment, a composition in which the content of the urethane (meth)acrylate is 90% by mass or more with respect to the total mass of the composition is also referred to as a "urethane (meth)acrylate composition".

The preferable range of the content of the urethane (meth)acrylate in the urethane (meth)acrylate composition in the second embodiment is the same as the preferable range of the content of the urethane (meth)acrylate in the urethane (meth)acrylate composition in the first embodiment.

The urethane (meth)acrylate composition of the second embodiment may contain other components such as a photopolymerization initiator.

<<Cured Product>>

The specific aspect of the method for performing photocuring using the photocurable composition of the second embodiment is the same as the specific aspect of the method for performing photocuring using the photocurable composition of the first embodiment.

< Stereolithography >

The photocurable composition of the second embodiment is preferably used for stereolithography.

Among them, the photocurable composition of the second embodiment can be suitably used for a molding method using a 3D printer.

In the second embodiment, "stereolithography" is one type of three-dimensional molding method using a 3D printer.

The details of specific aspects, preferable aspects, and the like of the method of stereolithography in the second embodiment are the same as the details of specific aspects, preferable aspects, and the like of the method of stereolithography in the first embodiment.

<Stereolithography Product>

The stereolithography product of the second embodiment is a stereolithography product of the photocurable composition of the second embodiment.

The stereolithography product of the second embodiment is excellent in antibacterial properties and biocompatibility.

(IC50)

In the stereolithography product of the second embodiment, the IC50 is preferably 5% or more.

It is considered that the higher the value of IC50 is, the more excellent biocompatibility is exhibited.

Since the stereolithography product of the second embodiment is the stereolithography product of the photocurable composition of the second embodiment, the value of the IC50 can be improved. Therefore, biocompatibility is excellent.

From the above viewpoint, in the stereolithography product of the second embodiment, the IC50 is more preferably 10% or more, still more preferably 30% or more, particularly preferably 50% or more, and further more preferably 70% or more.
The value of IC50 is measured by the method of ISO 10993-5: 2009 Annex B.

<3D Printer>

The details of specific examples, preferable specific examples, preferable aspects, and the like of the stereolithography system in the second embodiment are the same as the details of specific examples, preferable specific examples, preferable aspects, and the like of the stereolithography type in the first embodiment.

<<Dental Product>>

The dental product of the second embodiment includes the stereolithography product of the second embodiment.
The photocurable composition of the second embodiment is preferably used for manufacturing a dental product.
The photocurable composition of the second embodiment is more preferably used for manufacturing a dental product by stereolithography.
The dental product of the second embodiment preferably contains a cured product of the photocurable composition of the second embodiment.
The details of specific examples, preferable specific examples, preferable aspects, and the like of the dental product containing the cured product (that is, a stereolithography product) of the photocurable composition of the second embodiment are the same as the details of specific examples, preferable specific examples, preferable aspects, and the like of the dental product containing the cured product (that is, a stereolithography product) of the photocurable composition of the first embodiment.
The second embodiment also includes the following aspects.

<2-1> A composition containing urethane (meth)acrylate and hydroxypropyl acrylate, in which the content of the hydroxypropyl acrylate is 25,000 ppm by mass or less with respect to the total mass of the composition.
<2-2> The composition according to <2-1>, in which the content of the hydroxypropyl acrylate is 10 ppm by mass or more with respect to the total mass of the composition.
<2-3> The composition according to <2-1> or <2-2>, in which the content of the hydroxypropyl acrylate is 15,000 ppm by mass or less with respect to the total mass of the composition.
<2-4> The composition according to any one of <2-1> to <2-3>, in which the urethane (meth)acrylate contains a urethane (meth)acrylate having 2 or less functional groups.
<2-5> The composition according to any one of <2-1> to <2-4>, further containing a photopolymerization initiator.
<2-6> The composition according to <2-5>, further containing a (meth)acrylic monomer (D) other than the urethane (meth)acrylate and the hydroxypropyl acrylate.
<2-7> The composition according to <2-5> or <2-6>, in which IC50 of the cured product is 5% or more.
<2-8> The composition according to any one of <2-5> to <2-7>, in which the content of the bifunctional (meth)acrylate and the monofunctional (meth)acrylate is 80% by mass or more with respect to the total mass of the (meth)acrylate in the composition.
<2-9> The composition according to any one of <2-5> to <2-8>, in which the content of the urethane (meth)acrylate is 25% by mass or more with respect to the total mass of the composition.
<2-10> The composition according to any one of <2-1> to <2-4>, in which the content of the urethane (meth)acrylate is 95% by mass or more with respect to the total mass of the composition.
<2-11> The composition according to any one of <2-5> to <2-9>, which is used for stereolithography.
<2-12> The composition according to any one of <2-1> to <2-11>, which is used for manufacturing a dental product.
<2-13> A stereolithography product of the composition according to any one of <2-5> to <2-9>.
<2-14> The stereolithography product according to <2-13>, in which the IC50 is 5% or more.
<2-15> A dental product including the stereolithography product according to <2-13> or <2-14>.

## EXAMPLES

Hereinafter, examples of the first embodiment will be described, but the first embodiment is not limited by the following examples. Abbreviations of the compounds used in the examples of the first embodiment are shown below.
In this example, "ppm" means ppm by mass.
HEA: 2-hydroxyethyl acrylate

HEMA: 2-hydroxyethyl methacrylate
4HBA: 4-hydroxybutyl acrylate
TMHDI: 2,2,4-trimethylhexamethylene diisocyanate
IPDI: isophorone diisocyanate
PTMG: PTMG 1000 (manufactured by Mitsubishi Chemical Corporation)
DBTDL: dibutyltin dilaurate
MEHQ: 4-methoxyphenol

<Synthesis Example 1A> UA-L: TMHDI + HEA

In a 1 liter 4-necked flask equipped with a sufficiently dried stirring blade and thermometer, 372 g (3.20 mol) of HEA, 0.71 g (0.1% by mass with respect to the total mass of HEA and TMHDI) of DBTDL, and 0.35 g (0.05% by mass with respect to the total mass of HEA and TMHDI) of MEHQ were added, the mixture was stirred until the mixture became uniform, and then the temperature was raised to 60°C. Subsequently, 337 g (1.60 mol) of TMHDI was added dropwise over 1 hour. Since the internal temperature increased due to the reaction heat during the dropwise addition, the amount of dropwise addition was controlled to be 80°C or less. After dropping the whole amount, the reaction was carried out for 10 hours while the reaction temperature was maintained at 80°C. At this time, the progress of the reaction was followed by HPLC analysis to confirm the end point of the reaction. The product was discharged from the reactor to obtain 680 g of urethane acrylate (UA-L) represented by the following formula. The viscosity at 25°C was 7,100 mPa s.

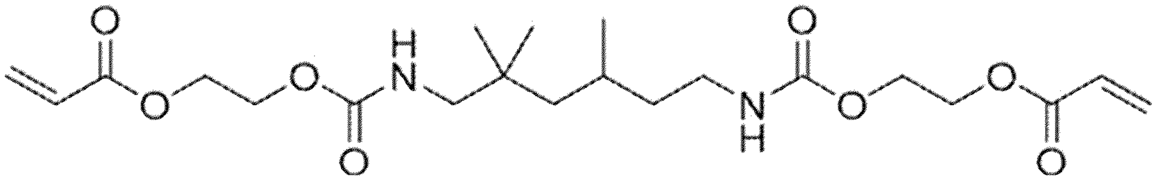

<Synthesis Example 2A> UA-M: IPDI + PTMG + HEA

In a 1 liter four-necked flask equipped with a sufficiently dried stirring blade and thermometer, 507 g (0.50 mol) of PTMG, 0.84 g (0.1% by mass with respect to the total mass of PTMG, 4HBA, and IPDI) of DBTDL, and 0.42 g (0.05% by mass with respect to the total mass of PTMG, 4HBA, and IPDI) of MEHQ were added, the mixture was stirred until the mixture became uniform, and then the temperature was raised to 60°C. Subsequently, 222 g (1.00 mol) of IPDI was added dropwise over 1 hour. Since the internal temperature increased due to the reaction heat during the dropwise addition, the amount of dropwise addition was controlled to be 80°C or less. After dropping the whole amount, the reaction was carried out for 10 hours while the reaction temperature was maintained at 80°C. At this time, the progress of the reaction was followed by HPLC analysis to confirm the end point of the reaction, and then 116 g (1.00 mol) of HEA was added dropwise over 1 hour. Since the internal temperature increased due to the reaction heat during the dropwise addition, the amount of dropwise addition was controlled to be 80°C or less. After dropping the whole amount, the reaction was carried out for 10 hours while the reaction temperature was maintained at 80°C. At this time, the progress of the reaction was followed by HPLC analysis to confirm the end point of the reaction. The product was discharged from the reactor to obtain 760 g of urethane acrylate (UA-M). The viscosity at 40°C was 25,000 mPa·s.

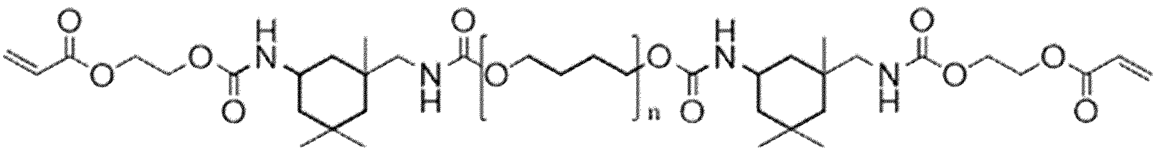

<Synthesis Example 3A> UA-N: TMHDI + HEMA

In a 1 liter 4-necked flask equipped with a sufficiently dried stirring blade and thermometer, 416 g (3.20 mol) of HEMA, 0.75 g (0.1% by mass with respect to the total mass of HEMA and TMHDI) of DBTDL, and 0.38 g (0.05% by mass with respect to the total mass of HEMA and TMHDI) of MEHQ were added, the mixture was stirred until the mixture became uniform, and then the temperature was raised to 60°C. Subsequently, 337 g (1.60 mol) of TMHDI was added dropwise over 1 hour. Since the internal temperature increased due to the reaction heat during the dropwise addition, the amount of dropwise addition was controlled to be 80°C or less. After dropping the whole amount, the reaction was carried out for 10 hours while the reaction temperature was maintained at 80°C. At this time, the progress of the reaction

was followed by HPLC analysis to confirm the end point of the reaction. The product was discharged from the reactor to obtain 720 g of urethane methacrylate (UA-N). The viscosity at 25°C was 8,200 mPa s.

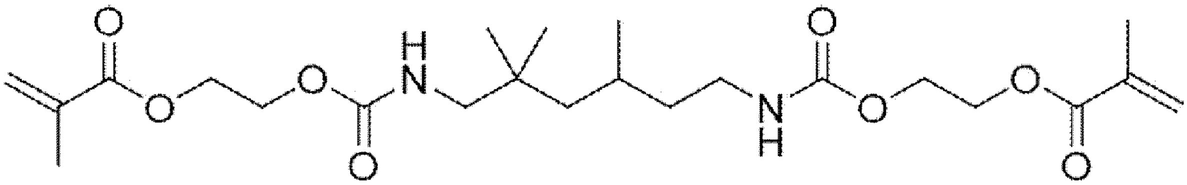

<Synthesis Example 4A> UA-O: TMHDI + 4HBA

In a 1 liter 4-necked flask equipped with a sufficiently dried stirring blade and thermometer, 461 g (3.20 mol) of 4HBA, 0.80 g (0.1% by mass with respect to the total mass of 4HBA and TMHDI) of DBTDL, and 0.40 g (0.05% by mass with respect to the total mass of 4HBA and TMHDI) of MEHQ were added, the mixture was stirred until the mixture became uniform, and then the temperature was raised to 60°C. Subsequently, 337 g (1.60 mol) of TMHDI was added dropwise over 1 hour. Since the internal temperature increased due to the reaction heat during the dropwise addition, the amount of dropwise addition was controlled to be 80°C or less. After dropping the whole amount, the reaction was carried out for 10 hours while the reaction temperature was maintained at 80°C. At this time, the progress of the reaction was followed by HPLC analysis to confirm the end point of the reaction. The product was discharged from the reactor to obtain 700 g of urethane acrylate (UA-O). The viscosity at 65°C was 270 mPa·s.

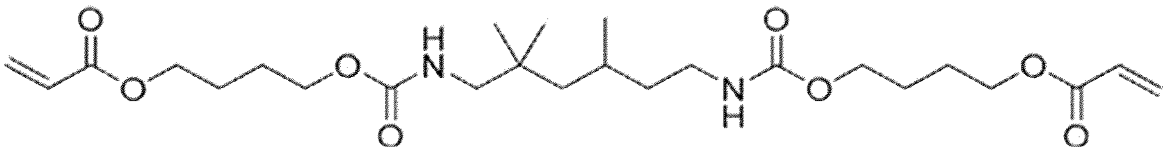

<Examples and Comparative Examples>

Urethane (meth)acrylate (UA-L, UA-M, UA-N, or UA-O) described in Table 1 and ethoxylated-o-phenylphenol acrylate (A-LEN-10, manufactured by Shin-Nakamura Chemical Co., Ltd.) were mixed at a mass ratio described in Table 1 to prepare a monomer composition. To 100 parts by mass of each monomer composition, 1 part by mass of 2,4,6-trimethylbenzoyl-diphenyl-phosphine oxide (Omnirad TPO (IGM resins)) as a photopolymerization initiator and 1 part by mass of 1-hydroxy-cyclohexyl-phenyl-ketone (Omnirad 184 (IGM resins)) were mixed to prepare a photocurable composition of each of the examples and comparative examples.

For the photocured compositions of Examples 1A to 5A and Comparative Example 1A, 2-hydroxyethyl acrylate (HEA) was further added such that the content thereof reaches ppm values as shown in Table 1.

The content of 2-hydroxyethyl acrylate (HEA) was confirmed by the following liquid chromatography mass spectrometry.

All the photocurable compositions of Examples 1A to 5A and Comparative Examples 1A to 3A had viscosities measured under the conditions of 25°C and 50 rpm by an E-type viscometer within the range of from 5 mPa·s to 20,000 mPa·s.

[Liquid Chromatography Mass Spectrometry]

The content of 2-hydroxyethyl acrylate (HEA) was measured by subjecting an appropriately diluted sample to HPLC and LC/MS. As HPLC, Acquity UPLC H-class system (manufactured by Waters) was used, and as LC/MS, Acquity UPLC H-class system/Xevo G2 Qtof (manufactured by Waters) was used.

[Biocompatibility]

Biocompatibility was evaluated using a 50% colony growth inhibition concentration IC50 as an index. It is considered that the higher the value of IC50 is, the more excellent biocompatibility is exhibited.

The evaluation criteria are as follows.

-Evaluation Criteria-

A: The value of IC50 was from 40 to 120.
B: The value of IC50 was from 5 to less than 40.
C: The value of IC50 was less than 5.

IC50 was measured according to ISO 10993-5: 2009 Annex B. Specifically, measurement and calculation were performed by the following method.

Using a 3D printer (Cara Print 4.0 manufactured by Kulzer Inc.), the obtained photocurable composition was irradiated with visible light having a wavelength of 405 nm at an irradiation amount of 12 mJ/cm$^2$ to form a cured layer P1 having a thickness of 100 $\mu$m, and the cured layer P1 was layered in a thickness direction to mold the photocurable composition to a size of 20 mm $\times$ 20 mm $\times$ 2 mm to obtain a molded article. The resulting molded article was immersed in isopropanol and washed for 5 minutes using an ultrasonic washer (KS-120 N manufactured by KYOWA IRIKA Co., Ltd.). The washed molded article was dried by air blowing, and then irradiated with ultraviolet rays having a wavelength of 365 nm under the condition of 10 J/cm$^2$ to be finally cured, thereby obtaining a stereolithography product. The obtained stereolithography product was washed with water for injection according to the Japanese Pharmacopoeia, and then irradiated with ultraviolet rays for 30 minutes on each of the front and back sides in a clean bench.

29 mg of L-glutamine, 11 mg of sodium pyruvate, 5 mg of kanamycin and 5 mL of fetal bovine serum were added to 100 mL of Eagle's minimum essential medium to prepare M05 medium. 13.3 mL of the M05 medium prepared for 10 stereolithography products after ultraviolet irradiation was added to a 50 mL hard glass beaker. The beaker was covered with an aluminum foil to be shielded from light, and allowed to stand in a 5% $CO_2$ incubator (BNS-110 manufactured by ESPEC CORP.) at 37°C for 24 hours, and then filtered to obtain an extract a. The extract a was defined as having a concentration of 100% by mass, and was appropriately diluted with the M05 medium to obtain test solutions a having concentrations of 3.13% by mass, 6.25% by mass, 12.5% by mass, 25% by mass, 50% by mass, and 100% by mass.

Only 10 mL of the M05 medium was collected in a 15 mL screw-capped light-shielded polypropylene bottle, allowed to stand in a 5% $CO_2$ incubator (BNS-110 manufactured by ESPEC CORP.) at 37°C for 24 hours, and then filtered to obtain an extract b. The extract b was defined as the test solution b.

The V79 cells (Chinese hamster) grown in a monolayer were detached by 0.05% by mass trypsin treatment, and a cell suspension at 100 cells/mL was prepared using M05 medium. 0.5 mL of the prepared cell suspension was seeded on each well of 24 holes of a plastic plate for tissue culture, and cultured in a 5% $CO_2$ incubator (BNS-110 manufactured by ESPEC CORP.) at 37°C for 6 hours. After the culturing, the medium in the well was replaced with 0.5 mL of the test solution, and the cells were further cultured for 6 days. For the test solution a, 5 wells were used for each photocurable resin composition, and for the test solution b, 3 wells were used.

After completion of the culture, the cells were fixed in a 10% by mass neutral buffered formalin solution and stained with a 0.1% by mass methylene blue solution. A colony in which approximately 50 or more cells were gathered was defined as one colony, and the stained colonies in each well were counted. Each concentration of the test solution a and the average number of colonies formed in the well using the test solution b were defined as n;(a) and n(b), respectively, and $r_i$ was defined for each concentration of the test solution a by the following Formula a.

$$r_i = \frac{(n(b) - n_i(a))}{n(b)}$$

i represents a concentration of a test solution a

(Formula a)

Using the calculated $r_i$, the 50% colony growth inhibition concentration IC50 value of the test solution a was

determined by the following Formula b.

$$\mathrm{IC50} = \exp\left(4.6052 - 0.6935 \times \left(0.5r_{3.13\%} + r_{6.25\%} + r_{12.5\%} + r_{25\%} + r_{50\%} + 0.5r_{100\%}\right)\right)$$

(Formula b)

[Antimicrobial Properties]

The obtained photocurable composition was molded to a size of 30 mm × 30 mm × 2 mm using a 3D printer (Cara Print 4.0 manufactured by Kulzer Inc.) to obtain a molded article. The resulting molded article was immersed in isopropanol and washed for 5 minutes using an ultrasonic washer (KS-120 N manufactured by KYOWA IRIKA Co., Ltd.). The washed molded article was dried by air blowing, and then irradiated with ultraviolet rays having a wavelength of 365 nm under the condition of 10 J/cm$^2$ to be finally cured, thereby obtaining a stereolithography product.

A glucose-added inorganic salt agar medium was aseptically dispensed into a petri dish such that the thickness of the medium was approximately 5 mm, and the medium was allowed to be cooled and solidified, thereby obtaining a glucose-added inorganic salt agar medium.

2.0 g of sodium nitrate, 0.7 g of potassium dihydrogen phosphate, 0.3 g of potassium dihydrogen phosphate, 0.5 g of potassium chloride, 0.5 g of magnesium sulfate heptahydrate, and 0.01 g of iron (II) sulfate heptahydrate were dissolved in 1000 mL of purified water, and the solution was prepared with a sterilized 0.01 mol/L sodium hydroxide aqueous solution such that the pH was 6.0 to 6.5 to obtain an inorganic salt solution.

5.0 mg of N-methyl taurine was added to 50 mL of the inorganic salt solution, and the mixture was sterilized with high-pressure steam at 121°C for 20 minutes to obtain a wet-added inorganic salt solution. SDL-321 manufactured by Tomy Seiko Co., Ltd. was used for high-pressure steam sterilization.

1.5 g of glucose was added to 50 mL of the inorganic salt solution, and the mixture was sterilized with high-pressure steam at 115°C for 30 minutes to obtain a glucose-added inorganic salt solution. SDL-321 manufactured by Tomy Seiko Co., Ltd. was used for high-pressure steam sterilization.

10 mL of a wet-added inorganic salt solution was added to a slant medium of Aspergillus niger in which spore formation was sufficiently observed, the surface spores were gently rubbed with a sterilized platinum wire, and the test tube was gently shaken to disperse the spores in the solution. Sterile glass beads were placed, the test tube was shaken and then filtered to remove hyphae. The filtrate was placed in a 15 mL centrifuge tube (AS ONE Corporation), and centrifuged at 1880 G for 5 minutes using CN-1040 manufactured by AS ONE Corporation. Then, the supernatant was discarded, and the residue was suspended in 25 mL of an inorganic salt solution and centrifuged again at 1880 G for 5 minutes. The supernatant was discarded and the residue was suspended in 50 mL of the inorganic salt solution. Using an improved Neubauer hemocytometer (manufactured by AS ONE Corporation), a glucose-added inorganic salt solution was added such that the number of spores per 1 mL of the suspension was approximately 106 to prepare a spore suspension.

The stereolithography product was horizontally placed on a glucose-added inorganic salt agar medium, and the stereolithography product and the surface of the medium were evenly sprayed with a spore suspension. The petri dish was capped and cultured at a temperature of 24°C and a humidity of 95% for 4 weeks.

The photograph of the stereolithography product after culture was analyzed with image analysis software, the area of the growth part of the hyphae was confirmed, and the antibacterial properties were evaluated according to the following criteria.

-Evaluation Criteria-

A: The area of the growth part of the hyphae is less than 25% of the area of the surface of the stereolithography product.

B: The area of the growth part of the hyphae is 25% or more and 50% or more of the area of the surface of the stereolithography product.

C: The area of the growth part of the hyphae is 50% or more of the area of the surface of the stereolithography product.

[Table 1]

| | Monomer composition ratio | | HEA [ppm] | Biocompatibility | Antibacterial properties |
|---|---|---|---|---|---|
| | Urethane (meth) acrylate (parts by mass) | (Meth)acrylic monomer (C) (parts by mass) | | | |
| Example 1A | UA-L (70) | X (30) | 7 | A | A |
| Example 2A | UA-L (30) | X (70) | 65 | A | A |
| Example 3A | UA-L (70) | X (30) | 152 | A | A |
| Example 4A | UA-M (50) | X (50) | 3021 | A | A |
| Example 5A | UA-L (70) | X (30) | 5012 | B | A |
| | UA-L | X | 11368 | C | A |
| Comparative Example 1A | (70) | (30) | | | |
| Comparative Example 2A | UA-N (70) | X (30) | 0 | A | C |
| Comparative Example 3A | UA-O (70) | X (30) | 0 | A | C |

As shown in Table 1, in Examples using a urethane (meth)acrylate composition containing a urethane (meth)acrylate and 2-hydroxyethyl acrylate, in which the content of 2-hydroxyethyl acrylate is 10,000 ppm by mass or less with respect to the total mass of the urethane (meth)acrylate composition, the evaluation of antibacterial properties was good, and the value of IC50 was high. Therefore, a stereolithography product having excellent antibacterial properties and biocompatibility could be obtained.

On the other hand, in Comparative Example 1A in which the content of 2-hydroxyethyl acrylate was not 10,000 ppm by mass or less with respect to the total mass of the urethane (meth)acrylate composition, the value of IC50 was low, and thus biocompatibility was poor.

In addition, Comparative Examples 2A and 3A not containing 2-hydroxyethyl acrylate were inferior in antibacterial properties.

Hereinafter, examples of the second embodiment will be described, but the second embodiment is not limited by the following examples. Abbreviations of the compounds used in the examples of the second embodiment are shown below.

In this example, "ppm" means ppm by mass.

HPA: Hydroxypropyl acrylate (Mixture of 2-hydroxypropyl acrylate and 2-hydroxy-1-methylethyl acrylate)

HEMA: 2-hydroxyethyl methacrylate
4HBA: 4-hydroxybutyl acrylate
TMHDI: 2,2,4-trimethylhexamethylene diisocyanate
IPDI: isophorone diisocyanate
PTMG: PTMG 1000 (manufactured by Mitsubishi Chemical Corporation)
DBTDL: dibutyltin dilaurate
MEHQ: 4-methoxyphenol

<Synthesis Example 1B> UA-P: TMHDI + HPA

In a 1 liter four-necked flask equipped with a sufficiently dried stirring blade and thermometer, 417 g (3.20 mol) of HPA, 0.75 g (0.1% by mass with respect to the total mass of HPA and TMHDI) of DBTDL, and 0.38 g (0.05% by mass with respect to the total mass of HPA and TMHDI) of MEHQ were added, and the mixture was stirred until the mixture became uniform, and then the temperature was raised to 60°C. Subsequently, 336 g (1.60 mol) of TMHDI was added dropwise over 1 hour. Since the internal temperature increased due to the reaction heat during the dropwise addition,

the amount of dropwise addition was controlled to be 80°C or less. After dropping the whole amount, the reaction was carried out for 10 hours while the reaction temperature was maintained at 80°C. At this time, the progress of the reaction was followed by HPLC analysis to confirm the end point of the reaction. The product was discharged from the reactor to obtain 720 g of urethane methacrylate (UA-P). The viscosity at 25°C was 8000 mPa·s.

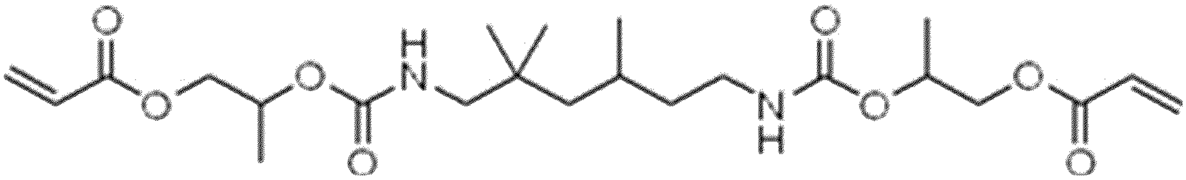

<Synthesis Example 2B> UA-Q: IPDI + PTMG + HPA

In a 1 liter four-necked flask equipped with a sufficiently dried stirring blade and thermometer, 507 g (0.50 mol) of PTMG, 0.84 g (0.1% by mass with respect to the total mass of PTMG, HPA, and IPDI) of DBTDL, and 0.43 g (0.05% by mass with respect to the total mass of PTMG, HPA, and IPDI) of MEHQ were added, the mixture was stirred until the mixture became uniform, and then the temperature was raised to 60°C. Subsequently, 222 g (1.00 mol) of IPDI was added dropwise over 1 hour. Since the internal temperature increased due to the reaction heat during the dropwise addition, the amount of dropwise addition was controlled to be 80°C or less. After dropping the whole amount, the reaction was carried out for 10 hours while the reaction temperature was maintained at 80°C. At this time, the progress of the reaction was followed by HPLC analysis to confirm the end point of the reaction, and then 130 g (1.00 mol) of HPA was added dropwise over 1 hour. Since the internal temperature increased due to the reaction heat during the dropwise addition, the amount of dropwise addition was controlled to be 80°C or less. After dropping the whole amount, the reaction was carried out for 10 hours while the reaction temperature was maintained at 80°C. At this time, the progress of the reaction was followed by HPLC analysis to confirm the end point of the reaction.

The product was discharged from the reactor to obtain 760 g of urethane acrylate (UA-Q). The viscosity at 40°C was 30,000 mPa s.

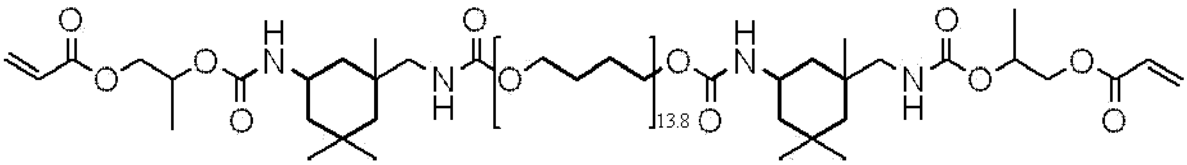

<Synthesis Example 3B> UA-N: TMHDI + HEMA

In a 1 liter 4-necked flask equipped with a sufficiently dried stirring blade and thermometer, 416 g (3.20 mol) of HEMA, 0.75 g (0.1% by mass with respect to the total mass of HEMA and TMHDI) of DBTDL, and 0.38 g (0.05% by mass with respect to the total mass of HEMA and TMHDI) of MEHQ were added, the mixture was stirred until the mixture became uniform, and then the temperature was raised to 60°C. Subsequently, 337 g (1.60 mol) of TMHDI was added dropwise over 1 hour. Since the internal temperature increased due to the reaction heat during the dropwise addition, the amount of dropwise addition was controlled to be 80°C or less. After dropping the whole amount, the reaction was carried out for 10 hours while the reaction temperature was maintained at 80°C. At this time, the progress of the reaction was followed by HPLC analysis to confirm the end point of the reaction. The product was discharged from the reactor to obtain 720 g of urethane methacrylate (UA-N). The viscosity at 25°C was 8,200 mPa s.

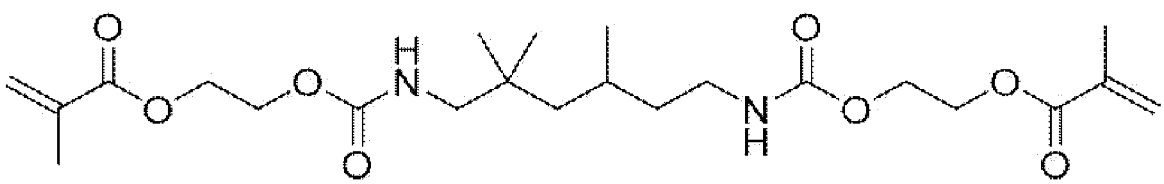

<Synthesis Example 4B> UA-O: TMHDI + 4HBA

In a 1 liter 4-necked flask equipped with a sufficiently dried stirring blade and thermometer, 461 g (3.20 mol)

of 4HBA, 0.80 g (0.1% by mass with respect to the total mass of 4HBA and TMHDI) of DBTDL, and 0.40 g (0.05% by mass with respect to the total mass of 4HBA and TMHDI) of MEHQ were added, the mixture was stirred until the mixture became uniform, and then the temperature was raised to 60°C. Subsequently, 337 g (1.60 mol) of TMHDI was added dropwise over 1 hour. Since the internal temperature increased due to the reaction heat during the dropwise addition, the amount of dropwise addition was controlled to be 80°C or less. After dropping the whole amount, the reaction was carried out for 10 hours while the reaction temperature was maintained at 80°C. At this time, the progress of the reaction was followed by HPLC analysis to confirm the end point of the reaction. The product was discharged from the reactor to obtain 700 g of urethane acrylate (UA-O). The viscosity at 65°C was 270 mPa·s.

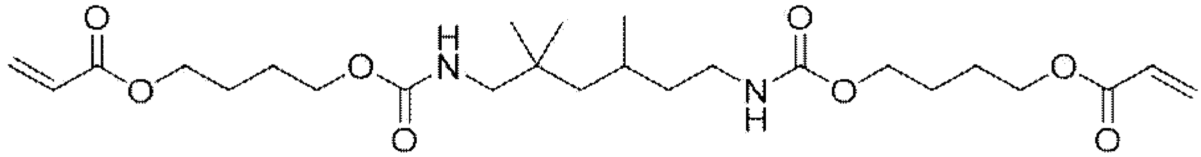

<Examples and Comparative Examples>

Urethane (meth)acrylate (UA-P, UA-Q, UA-N, or UA-O) described in Table 2 and ethoxylated-o-phenylphenol acrylate (A-LEN-10, manufactured by Shin-Nakamura Chemical Co., Ltd., also referred to as "X") were mixed at a mass ratio described in Table 2 to prepare a monomer composition.
To 100 parts by mass of each monomer composition, 1 part by mass of 2,4,6-trimethylbenzoyl-diphenyl-phosphine oxide (Omnirad TPO (IGM resins)) as a photopolymerization initiator and 1 part by mass of 1-hydroxy-cyclohexyl-phenyl-ketone (Omnirad 184 (IGM resins)) were mixed to prepare a photocurable composition of each of the examples and comparative examples.
For the photocured compositions of Examples 1B to 4B and Comparative Example 1B, hydroxypropyl acrylate (HPA) was further added such that the content thereof reaches ppm values as shown in Table 2.
The content of hydroxypropyl acrylate (HPA) was confirmed by the following liquid chromatography mass spectrometry.
All the photocurable compositions of Examples 1B to 4B and Comparative Examples 1B to 3B had viscosities measured under the conditions of 25°C and 50 rpm by an E-type viscometer within the range of from 5 mPa·s to 20,000 mPa·s.

[Liquid Chromatography Mass Spectrometry]

The content of hydroxypropyl acrylate (HPA) was measured by subjecting an appropriately diluted sample to HPLC and LC/MS. As HPLC, Acquity UPLC H-class system (manufactured by Waters) was used, and as LC/MS, Acquity UPLC H-class system/Xevo G2 Qtof (manufactured by Waters) was used.

[Biocompatibility]

Biocompatibility was evaluated using a 50% colony growth inhibition concentration IC50 as an index. It is considered that the higher the value of IC50 is, the more excellent biocompatibility is exhibited.
When the value of IC50 is, for example, 5% or more, biocompatibility is considered to be good, and when the value is 40% or more, biocompatibility is considered to be better.
The evaluation criteria are as follows.

-Evaluation Criteria-

A: The value of IC50 was from 40 to 120.
B: The value of IC50 was from 5 to less than 40.
C: The value of IC50 was less than 5.

IC50 was measured according to ISO 10993-5: 2009 Annex B. Specifically, measurement and calculation were performed by the following method.
Using a 3D printer (Cara Print 4.0 manufactured by Kulzer Inc.), the obtained photocurable composition was

irradiated with visible light having a wavelength of 405 nm at an irradiation amount of 12 mJ/$cm^2$ to form a cured layer P1 having a thickness of 100 μm, and the cured layer P1 was layered in a thickness direction to mold the photocurable composition to a size of 20 mm × 20 mm × 2 mm to obtain a molded article. The resulting molded article was immersed in isopropanol and washed for 5 minutes using an ultrasonic washer (KS-120 N manufactured by KYOWA IRIKA Co., Ltd.). The washed molded article was dried by air blowing, and then irradiated with ultraviolet rays having a wavelength of 365 nm under the condition of 10 J/$cm^2$ to be finally cured, thereby obtaining a stereolithography product.

The obtained stereolithography product was washed with water for injection according to the Japanese Pharmacopoeia, and then irradiated with ultraviolet rays for 30 minutes on each of the front and back sides in a clean bench.

29 mg of L-glutamine, 11 mg of sodium pyruvate, 5 mg of kanamycin and 5 mL of fetal bovine serum were added to 100 mL of Eagle's minimum essential medium to prepare M05 medium. 13.3 mL of the M05 medium prepared for 10 stereolithography products after ultraviolet irradiation was added to a 50 mL hard glass beaker. The beaker was covered with an aluminum foil to be shielded from light, and allowed to stand in a 5% $CO_2$ incubator (BNS-110 manufactured by ESPEC CORP.) at 37°C for 24 hours, and then filtered to obtain an extract a. The extract a was defined as having a concentration of 100% by mass, and was appropriately diluted with the M05 medium to obtain test solutions a having concentrations of 3.13% by mass, 6.25% by mass, 12.5% by mass, 25% by mass, 50% by mass, and 100% by mass.

Only 10 mL of the M05 medium was collected in a 15 mL screw-capped light-shielded polypropylene bottle, allowed to stand in a 5% $CO_2$ incubator (BNS-110 manufactured by ESPEC CORP.) at 37°C for 24 hours, and then filtered to obtain an extract b.

The extract b was defined as the test solution b.

The V79 cells (Chinese hamster) grown in a monolayer were detached by 0.05% by mass trypsin treatment, and a cell suspension at 100 cells/mL was prepared using M05 medium. 0.5 mL of the prepared cell suspension was seeded on each well of 24 holes of a plastic plate for tissue culture, and cultured in a 5% $CO_2$ incubator (BNS-110 manufactured by ESPEC CORP.) at 37°C for 6 hours. After the culturing, the medium in the well was replaced with 0.5 mL of the test solution, and the cells were further cultured for 6 days. For the test solution a, 5 wells were used for each photocurable resin composition, and for the test solution b, 3 wells were used.

After completion of the culture, the cells were fixed in a 10% by mass neutral buffered formalin solution and stained with a 0.1% by mass methylene blue solution. A colony in which approximately 50 or more cells were gathered was defined as one colony, and the stained colonies in each well were counted. Each concentration of the test solution a and the average number of colonies formed in the well using the test solution b were defined as n;(a) and n(b), respectively, and $r_i$ was defined for each concentration of the test solution a by the following Formula a.

$$r_i = \frac{(n(b) - n_i(a))}{n(b)}$$

i represents a concentration of a test solution a

(Formula a)

Using the calculated $r_i$, the 50% colony growth inhibition concentration IC50 value of the test solution a was determined by the following Formula b.

$$IC50 = \exp(4.6052 - 0.6935 \times (0.5r_{3.13\%} + r_{6.25\%} + r_{12.5\%} + r_{25\%} + r_{50\%} + 0.5r_{100\%}))$$

(Formula b)

[Antimicrobial Properties]

The obtained photocurable composition was molded to a size of 30 mm × 30 mm × 2 mm using a 3D printer (Cara Print 4.0 manufactured by Kulzer Inc.) to obtain a molded article. The resulting molded article was immersed in isopropanol and washed for 5 minutes using an ultrasonic washer (KS-120 N manufactured by KYOWA IRIKA Co., Ltd.). The washed molded article was dried by air blowing, and then irradiated with ultraviolet rays having a wavelength of 365 nm under the condition of 10 J/$cm^2$ to be finally cured, thereby obtaining a stereolithography product.

A glucose-added inorganic salt agar medium was aseptically dispensed into a petri dish such that the thickness of the medium was approximately 5 mm, and the medium was allowed to be cooled and solidified, thereby obtaining a glucose-added inorganic salt agar medium.

2.0 g of sodium nitrate, 0.7 g of potassium dihydrogen phosphate, 0.3 g of potassium dihydrogen phosphate, 0.5 g of potassium chloride, 0.5 g of magnesium sulfate heptahydrate, and 0.01 g of iron (II) sulfate heptahydrate were dissolved in 1000 mL of purified water, and the solution was prepared with a sterilized 0.01 mol/L sodium hydroxide aqueous solution such that the pH was 6.0 to 6.5 to obtain an inorganic salt solution.

5.0 mg of N-methyl taurine was added to 50 mL of the inorganic salt solution, and the mixture was sterilized with high-pressure steam at 121°C for 20 minutes to obtain a wet-added inorganic salt solution. SDL-321 manufactured by Tomy Seiko Co., Ltd. was used for high-pressure steam sterilization.

1.5 g of glucose was added to 50 mL of the inorganic salt solution, and the mixture was sterilized with high-pressure steam at 115°C for 30 minutes to obtain a glucose-added inorganic salt solution. SDL-321 manufactured by Tomy Seiko Co., Ltd. was used for high-pressure steam sterilization.

10 mL of a wet-added inorganic salt solution was added to a slant medium of Aspergillus niger in which spore formation was sufficiently observed, the surface spores were gently rubbed with a sterilized platinum wire, and the test tube was gently shaken to disperse the spores in the solution. Sterile glass beads were placed, the test tube was shaken and then filtered to remove hyphae. The filtrate was placed in a 15 mL centrifuge tube (AS ONE Corporation), and centrifuged at 1880 G for 5 minutes using CN-1040 manufactured by AS ONE Corporation. Then, the supernatant was discarded, and the residue was suspended in 25 mL of an inorganic salt solution and centrifuged again at 1880 G for 5 minutes. The supernatant was discarded and the residue was suspended in 50 mL of the inorganic salt solution. Using an improved Neubauer hemocytometer (manufactured by AS ONE Corporation), a glucose-added inorganic salt solution was added such that the number of spores per 1 mL of the suspension was approximately 106 to prepare a spore suspension.

The stereolithography product was horizontally placed on a glucose-added inorganic salt agar medium, and the stereolithography product and the surface of the medium were evenly sprayed with a spore suspension. The petri dish was capped and cultured at a temperature of 24°C and a humidity of 95% for 4 weeks.

The photograph of the stereolithography product after culture was analyzed with image analysis software, the area of the growth part of the hyphae was confirmed, and the antibacterial properties were evaluated according to the following criteria.

-Evaluation Criteria-

A: The area of the growth part of the hyphae is less than 25% of the area of the surface of the stereolithography product.

B: The area of the growth part of the hyphae is from 25% to less than 50% of the area of the surface of the stereolithography product.

C: The area of the growth part of the hyphae is 50% or more of the area of the surface of the stereolithography product.

[Table 2]

| | Monomer composition ratio | | HPA [ppm] | Biocompatibility | Antibacterial properties |
|---|---|---|---|---|---|
| | Urethane (meth) acrylate (parts by mass) | (Meth)acrylic monomer (D) (parts by mass) | | | |
| Example 1B | UA-P (70) | X (30) | 46 | A | A |
| Example 2B | UA-P (30) | X (70) | 381 | A | A |
| Example 3B | UA-P (70) | X (30) | 12650 | A | A |
| Example 4B | UA-Q (50) | X (50) | 20432 | B | A |
| Comparative Example 1B | UA-P (70) | X (30) | 31184 | C | A |
| | UA-N | X | 0 | A | C |
| Comparative Example 2B | (70) | (30) | | | |
| Comparative Example 3B | UA-O (70) | X (30) | 0 | A | C |

As shown in Table 2, in Examples using a urethane (meth)acrylate composition containing a urethane (meth)acrylate and hydroxypropyl acrylate, in which the content of hydroxypropyl acrylate is 25,000 ppm by mass or less with respect to the total mass of the composition, the evaluation of antibacterial properties was good, and the value of IC50 was high. Therefore, a stereolithography product having excellent antibacterial properties and biocompatibility could be obtained.

In particular, in Examples 1B to 3B using the urethane (meth)acrylate composition in which the content of hydroxypropyl acrylate is 15,000 ppm by mass or less with respect to the total mass of the urethane (meth)acrylate composition, the IC50 was 40% or more, and the biocompatibility was particularly high.

On the other hand, in Comparative Example 1B in which the content of hydroxypropyl acrylate was not 25,000 ppm by mass or less with respect to the total mass of the urethane (meth)acrylate composition, the value of IC50 was extremely low, and thus biocompatibility was poor.

In addition, Comparative Examples 2B and 3B not containing hydroxypropyl acrylate were inferior in antibacterial properties.

The disclosures of Japanese Patent Application No. 2021-049004 filed on March 23, 2021 and Japanese Patent Application No. 2022-003891 filed on January 13, 2022 are incorporated herein by reference in their entirety.

All publications, patent applications, and technical standards mentioned herein are incorporated in the present specification to the same extent as if each individual publication, patent application, and technical standard were specifically and individually noted to be incorporated by reference.

## Claims

**1.** A composition comprising:

a urethane (meth)acrylate, and
at least one selected from the group consisting of 2-hydroxyethyl acrylate and hydroxypropyl acrylate,
wherein at least one of the following (1) or (2) is satisfied:

(1) a content of 2-hydroxyethyl acrylate is 10,000 ppm by mass or less with respect to a total mass of the composition;
(2) a content of hydroxypropyl acrylate is 25,000 ppm by mass or less with respect to the total mass of the composition.

**2.** The composition according to claim 1, wherein the content of 2-hydroxyethyl acrylate is 5 ppm by mass or more with respect to the total mass of the composition.

3. The composition according to claim 1 or 2, wherein the content of 2-hydroxyethyl acrylate is 4,000 ppm by mass or less with respect to the total mass of the composition.

4. The composition according to any one of claims 1 to 3, wherein the content of hydroxypropyl acrylate is 10 ppm by mass or more with respect to the total mass of the composition.

5. The composition according to any one of claims 1 to 4, wherein the content of hydroxypropyl acrylate is 15,000 ppm by mass or less with respect to the total mass of the composition.

6. The composition according to any one of claims 1 to 5, wherein the urethane (meth)acrylate contains a urethane (meth)acrylate having 2 or less functional groups.

7. The composition according to any one of claims 1 to 6, further comprising a photopolymerization initiator.

8. The composition according to claim 7, further comprising a (meth)acrylic monomer (E) other than the urethane (meth)acrylate, the 2-hydroxyethyl acrylate, and the hydroxypropyl acrylate.

9. The composition according to claim 7 or 8, wherein IC50 of a cured product of the composition is 5% or more.

10. The composition according to any one of claims 7 to 9, wherein a content of a bifunctional (meth)acrylate and a monofunctional (meth)acrylate is 80% by mass or more with respect to a total mass of (meth)acrylate in the composition.

11. The composition according to any one of claims 7 to 10, wherein a content of the urethane (meth)acrylate is 25% by mass or more with respect to the total mass of the composition.

12. The composition according to any one of claims 1 to 6, wherein a content of the urethane (meth)acrylate is 95% by mass or more with respect to the total mass of the composition.

13. The composition according to any one of claims 7 to 11, which is used for stereolithography.

14. The composition according to any one of claims 1 to 13, which is used in manufacturing a dental product.

15. A stereolithography product of the composition according to any one of claims 7 to 11.

16. The stereolithography product according to claim 15, wherein IC50 is 5% or more.

17. A dental product comprising the stereolithography product according to claim 15 or 16.

## INTERNATIONAL SEARCH REPORT

International application No.
**PCT/JP2022/010410**

**A. CLASSIFICATION OF SUBJECT MATTER**

***C08F 290/00***(2006.01)i; ***B33Y 10/00***(2015.01)i; ***B33Y 80/00***(2015.01)i; ***B29C 64/112***(2017.01)i; ***B29C 64/124***(2017.01)i; ***B29C 64/314***(2017.01)i; ***B29C 64/343***(2017.01)i; ***B33Y 40/00***(2020.01)i; ***B33Y 70/00***(2020.01)i
FI: C08F290/00; B29C64/314; B29C64/343; B33Y70/00; B33Y40/00; B33Y80/00; B29C64/124; B29C64/112; B33Y10/00

According to International Patent Classification (IPC) or to both national classification and IPC

**B. FIELDS SEARCHED**

Minimum documentation searched (classification system followed by classification symbols)

C08F290/00; B33Y10/00; B33Y80/00; B29C64/112; B29C64/124; B29C64/314; B29C64/343; B33Y40/00; B33Y70/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2003-119231 A (MITSUBISHI RAYON CO., LTD.) 23 April 2003 (2003-04-23) claims, paragraphs [0009], [0020], examples | 1-13, 15, 16 |
| X | JP 2020-84093 A (KJ CHEMICALS CORP.) 04 June 2020 (2020-06-04) claims, paragraph [0063], examples | 1-13, 15, 16 |
| X | JP 2007-30479 A (MITSUBISHI CHEMICALS CORP.) 08 February 2007 (2007-02-08) claims, examples | 1-13, 15, 16 |
| X | WO 2017/047615 A1 (KJ CHEMICALS CORP.) 23 March 2017 (2017-03-23) claims, examples | 1-13, 15, 16 |
| X | JP 61-186303 A (SHOFU INC.) 20 August 1986 (1986-08-20) claims, examples | 1-17 |
| A | JP 2003-96146 A (MITSUBISHI RAYON KK) 03 April 2003 (2003-04-03) claims, examples | 1-17 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **11 May 2022** | **24 May 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/010410**

| Patent document cited in search report | Publication date (day/month/year) | Patent family member(s) | Publication date (day/month/year) |
|---|---|---|---|
| JP 2003-119231 A | 23 April 2003 | (Family: none) | |
| JP 2020-84093 A | 04 June 2020 | (Family: none) | |
| JP 2007-30479 A | 08 February 2007 | (Family: none) | |
| WO 2017/047615 A1 | 23 March 2017 | US 2018/0244831 A1<br>claims, examples<br>EP 3351575 A1<br>KR 10-2018-0054725 A<br>CN 108350141 A<br>TW 201720850 A | |
| JP 61-186303 A | 20 August 1986 | (Family: none) | |
| JP 2003-96146 A | 03 April 2003 | US 2004/0013976 A1<br>claims, examples<br>WO 2002/044285 A1<br>EP 1350816 A1<br>AT 469949 T | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 4160311 B
- JP 5111880 B
- JP 5235056 B
- JP 2021049004 A
- JP 2022003891 A